# EUROPEAN PATENT APPLICATION

(11) **EP 1 167 342 A1**
(43) Date of publication of application: **02.01.2002**
(21) Application number: 01305391.3
(22) Date of filing: 21.06.2001
(51) Int. Cl.: C07C 205/46, A61K 31/12, A61K 31/395, A61P 25/00, C07C 225/16, C07D 295/10, C07C 323/22, C07D 217/04, C07D 211/62, C07D 211/14, C07D 211/64, C07D 213/74, C07D 239/42, C07D 295/18

(54) **Novel substituted nitrocatechols, their use in the treatment of some central and peripheral nervous system disorders and pharmaceutical compositions containing them**

(30) Priority: 21.06.2000 GB 0015225
(71) Applicant: Portela & Ca., S.A., 4745-457 S. Mamede do Coronado (PT)
(72) Inventor: Learmonth, David Alexander, Vermoin, 4470 Maia (PT); Soares da Silva, Patricio Manuel Vieira Araujo, 4150 Porto (PT)
(74) Representative: Curtis, Philip Anthony

(57) **Abstract**

New compounds of formula I are described:

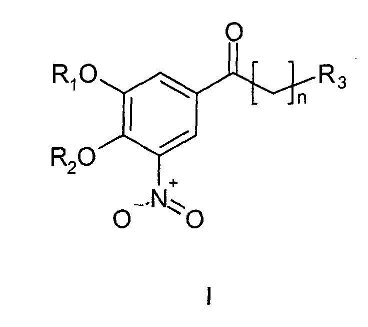

The compounds have potentially valuable pharmaceutical properties in the treatment of some central and peripheral nervous system disorders.

## Description

In recent years, the development of new inhibitors of the enzyme catechol-*O*-methyl transferase (COMT) has been accelerated by the hypothesis that inhibition of this enzyme may provide significant clinical improvements in patients afflicted by Parkinson's disease undergoing treatment with L-DOPA plus a peripheral AADC inhibitor. The rationale for the use of COMT inhibitors is based on their capacity to inhibit the *O*-methylation of L-DOPA to 3-*O*-methyl-L-Dopa. COMT inhibition slows elimination of L-DOPA from the plasma by increasing plasma half-life (increases area under the curve [AUC] without altering the time L-DOPA plasma to peak or the maximum concentration). Thus pharmacokinetic alterations may be an advantage over increasing the dose of L-DOPA, which also increases AUC, but additionally raises peak concentrations. With repeated doses of L-DOPA every 2-6 h in the presence of COMT inhibition, the mean plasma L-DOPA concentration is raised and the through concentrations are increased proportionally more than the peak concentrations despite a reduction in L-DOPA dose. As would be predicted by the slowed elimination of L-DOPA, the duration of antiparkinsonian action with single doses of L-DOPA is prolonged by COMT inhibition (Nutt, J.G., Lancet, 351:1221-1222, 1998). The most potent COMT inhibitors thusfar reported, 3,4-dihydroxy-4'-methyl-5-nitrobenzophenone (tolcapone, Australian Pat. AU-B-69764/87), and (E)-2-cyano-N,N-diethyl-3-(3,4-dihydroxy-5-nitrophenyl)acrylamide (entacapone, German Pat. DE 3740383 A 1) have inhibition constants in the low nM range. Tolcapone differs from entacapone in being a more potent inhibitor of COMT in the periphery and furthermore at penetrating into the brain to inhibit brain COMT as well. Due to unacceptable liver toxicity, only entacapone is currently used for the treatment of patients afflicted with Parkinson's disease undergoing treatment with L-DOPA plus a peripheral AADC inhibitor. Compounds penetrating the blood-brain barrier may be assumed to be more effective as theoretically they might have additional benefits of decreasing dopamine methylation to 3-methoxytyramine and homovanillic acid. Conversely, central inhibition may be unimportant if the more significant action is to protect L-DOPA from breakdown in the periphery. This distinction may have practical importance, as the use of COMT inhibitors which are excluded from the brain may avoid potential undesired CNS side effects of these agents.

We have discovered that heteroatom substitution at the alpha-carbon relative to the carbonyl moiety of the 3,4-dihydroxy-5-nitrobenzoyl group pharmacophore gives rise to compounds possessing pronounced effects of potential usefulness for COMT inhibition. The invention relates to compounds of general formula I; 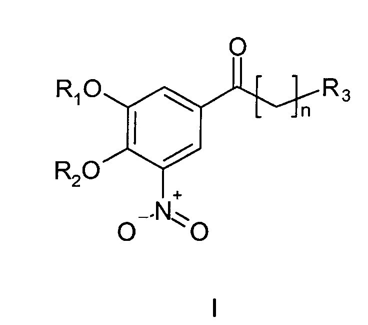 where R₁ and R₂ are the same or different and signify hydrogens or groups hydrolysable under physiological conditions, optionally substituted lower alkanoyl or aroyl, optionally substituted lower alkyl or arylsulphonyl or optionally substituted lower alkylcarbamoyl or taken together signify a lower alkylidene or cycloalkylidene group; n signifies the number 1 or 2; R₃ represents the group -O-R₄ wherein R₄ signifies an aryl group or R₃ represents the group -S-R₅ wherein R₅ signifies an aryl or heteroaryl group or R₃ represents the group -NH-R₆ wherein R₆ signifies alkyl, cycloalkyl, heterocycloalkyl, alkylaryl, aryl, heteroaryl or alkylcarbonyl group; or R₃ represents the group -CHR₄NR₇ wherein NR₇ signifies a heterocycloalkyl group connected via the ring nitrogen atom; or R₃ represents the group NR₈R₉ where R₈ and R₉ signify alkyl groups, or taken together form a pyridyl, phthalimido or a heterocycloalkyl group connected via the ring nitrogen atom; or R₃ signifies a piperidine or piperazine ring optionally substituted by alkyl, alkylaryl, heterocycloalkyl, aryl, heteroaryl, alkyloxy, alkoxycarbonyl, alkylcarbonyl, cyano, carbamoyl or N-lower alkyl substituted or disubstituted carbamoyl group; the term alkyl means carbon chains, straight or branched, containing from one to six carbon atoms, optionally substituted by alkoxy, halogen, alkoxycarbonyl or hydroxycarbonyl groups; the term cycloalkyl represents an alicyclic group with three to six carbon atoms; the term aryl means a phenyl or naphthyl group, optionally substituted by alkoxy, halogen, or nitro groups; the term heteroaryl represents a five or six-membered ring, incorporating an atom of oxygen, sulphur or nitrogen; the term heterocycloalkyl represents a four to eight-membered cyclic ring optionally incorporating other atoms of oxygen, sulphur or nitrogen; the term halogen represents fluorine, chlorine, bromine or iodine and to the pharmacologically acceptable salts thereof; to the use of the compounds for prevention or treatment of certain pathological states in humans, and to preparation of pharmaceutical compositions containing them.

For the preparation of pharmaceutical compositions of compounds of formula I, inert pharmaceutically acceptable carriers are admixed with the active compounds. The pharmaceutically acceptable carriers may be either solid or liquid. Solid form preparations include powders, tablets, dispersible granules and capsules. A solid carrier can be one or more substances which may also act as diluents, flavouring agents, solubilizers, lubricants, suspending agents, binders or tablet disintegrating agents; it may also be an encapsulating material.

Preferably, the pharmaceutical preparation is in unit dosage form, e.g. packaged preparation, the package containing discrete quantities of preparation such as packeted tablets, capsules and powders in vials or ampoules.

The dosages may be varied depending on the requirement of the patient, the severity of the disease and the particular compound being employed. For convenience, the total daily dosage may be divided and administered in portions throughout the day. Determination of the proper dosage for a particular situation is within the skill of those in the medical art.

### Material and Methods

### Studies in Human neuroblastoma cells

SK-N-SH cells (ATCC HTB-11) were obtained from the American Type Culture Collection and maintained in a humidified atmosphere of 5% CO₂-95% air at 37°C. The cells were grown in Minimal Essential Medium supplemented with 10% foetal bovine serum, 100 U/ml penicillin G, 0.25 µg/ml amphotericin B, 100 µg/ml streptomycin, and 25 mM *N*-2-hydroxyethylpiperazine-*N*'-2-ethanosulfonic acid (HEPES). For subculturing, the cells were dissociated with 0.05% trypsin-EDTA, split 1:4 and subcultured in Costar flasks with 21-cm² growth areas (Costar, Badhoevedorp, The Netherlands). For *O*-methylation studies, the cells were seeded in 96 well plates and 24 hours prior to each experiment the medium was changed to medium free of foetal bovine serum. The cell medium was changed every two days and experiments were generally performed after cells reached confluence (5-7 days) and each cm² contained about 100 µg of cell protein. On the day of the experiment, the growth medium was aspirated and the cells washed with phosphate buffer (5 mM). COMT activity was evaluated in cell monolayers by the ability to methylate adrenaline (0.03 to 100 µM) to metanephrine in the presence of saturating concentration of the S-adenosyl-L-methionine, the methyl donor (250 µM), pargyline (100 µM), MgCl₂ (100 µM) and EGTA (1 mM). The preincubation and incubation were carried out at 37°C under conditions of light protection with continuous shaking and without oxygenation. After preincubation, cells were incubated for 15 min with 1000 µM epinephrine. The reaction was terminated by the addition of 15 µl of 2 M perchloric acid. The acidified samples were stored at 4°C before injection into the high pressure liquid chromatograph for the assay of metanephrine. The assay of metanephrine was carried out by means of high pressure liquid chromatography with electrochemical detection. The lower limits for detection of metanephrine ranged from 350 to 500 fmol (0.5 to 1.0 pmol/mg protein/h).

Kₘ and Vₘₐₓ values for COMT activity were calculated from non-linear regression analysis using the GraphPad Prism statistics software package (Motulsky, H.G., et al., GraphPad Prisms, GraphPad Prism Software Inc., San Diego, 1994). For the calculation of the IC₅₀ values, the parameters of the equation for one site inhibition were fitted to the experimental data. Geometric means are given with 95% confidence limits and arithmetic means are given with S.E.M.. Statistical analysis was performed by one-way analysis of variance (ANOVA) using Newman-Keuls multiple comparison test to compare values.

The protein content in the homogenates was determined by the method of Bradford (Bradford, M.M., Anal. Biochem., 72: 248-254, 1976) with human serum albumin as standard. The protein content was similar in all samples (approximately 2 mg/500 µl homogenate).

### Studies in mice tissues

Tissues (liver and brain) from NMRI mice weighing 25-30 g (Harlan-Interfauna Ibérica, Barcelona, Spain) kept ten per cage under controlled environmental conditions (12 h light/dark cycle and room temperature 24 °C), were used in all experiments. Tissues, obtained from pentobarbitone (60 mg/Kg) anaesthetised mice, were used in the experiments. Tissues were immediately removed and homogenised in 5 mM phosphate buffer, pH 7.8 and stored at -80°C.

COMT activity was evaluated by the ability to methylate adrenaline to metanephrine, as previously described (Vieira-Coelho, M.A., Soares-da-Silva, P., Brain Res, 1999, 821,69-78). Aliquots of 0.5 ml of liver and brain homogenates were preincubated for 20 min with 0.4 ml of phosphate buffer (5 mM); thereafter, the reaction mixture was incubated for 10 min with increasing concentrations of adrenaline (0.1 to 100 µM; 0.1 ml) in the presence of a saturating concentration of S-adenosyl-L-methionine, the methyl donor (250 µM). The incubation medium also contained pargyline (100 µM), MgCl₂ (100 µM) and EGTA (1 mM). The preincubation and incubation were carried out at 37°C under conditions of light protection with continuous shaking and without oxygenation.

In experiments designed to evaluate the oral bioavailability, half-life and brain access, test compounds (in saline with 10 % tween 80) were given by gastric tube to overnight fasted mice. Thereafter, at defined intervals, livers and brains were removed and used to determine COMT activity in the presence of a concentration of adrenaline five times the corresponding Kₘ value as determined in saturation experiments. At the end of the incubation period the tubes were transferred to ice and the reaction was stopped by the addition of 200 µl of 2 M perchloric acid. The samples were then centrifuged (200x*g*, 4 min, 4°C), and 500 µl aliquots of the supernatant, filtered on 0.22 µm pore size Spin-X filter tubes (Costar) were used for the assay of metanephrine.

### Results

### In vitro COMT inhibition studies

Incubation of SK-N-SH cells in the presence of increasing concentrations of adrenaline resulted in a concentration-dependent formation of metanephrine, yielding Kₘ (in µM) and Vₘₐₓ (in nmol mg protein⁻¹ h⁻¹) values of 1.1±0.4 and 2.7±0.1, respectively. From these kinetic parameters, a saturating concentration of adrenaline was chosen to use in inhibition studies (adrenaline = 50 µM). Compounds of formulae 1 - 30 and entacapone (the reference compound) produced marked decreases in the O-methylation of adrenaline in intact SK-N-SH cells (see table 1).

### In vitro ex vivo COMT inhibition studies

Incubation of liver and whole brain homogenates in the presence of increasing concentrations of adrenaline resulted in a concentration-dependent formation of metanephrine, yielding Kₘ (in µM) and Vₘₐₓ (in nmol mg protein⁻¹ h⁻¹) values of 1.6±0.4 and 1.20±0.05 for brain and 5.1±1.9 and 24.9±2.1 for liver, respectively. From these kinetic parameters, a saturating concentration of adrenaline was chosen to use in inhibition studies (adrenaline = 50 µM). Compounds 1 - 30 were found to be potent inhibitors of liver COMT, the maximal inhibitory effect being achieved within 1 h after their oral administration (table 2). Compounds **2, 3, 10** and **18** were much more potent upon liver COMT than brain COMT. On the other hand, Compounds **5** and **16** were equally potent in inhibiting liver and brain COMT activity.

**Table 1.**

| Effect of compounds 1 - 30 and entacapone upon COMT activity in SK-N-SH cells. The concentration of test compound was 100 nM. Results are means ±SEM of n=4-8. | | |
|---|---|---|
| | Compounds | COMT activity (% of control) |
| 1 | BIA 3-257 | 4.3±0.6 |
| 2 | BIA 3-260 | 7.5±0.6 |
| 3 | BIA 3-282 | 7.2±1.9 |
| 4 | BIA 3-283 | 23.8±2.9 |
| 5 | BIA 3-284 | 40.2±8.7 |
| 6 | BIA 3-286 | 12.2±1.7 |
| 7 | BIA 3-294 | 9.0±0.2 |
| 8 | BIA 3-303 | 8.6±0.1 |
| 9 | BIA 3-304 | 12.8±0.4 |
| 10 | BIA 3-309 | 2.0±0.1 |
| 11 | BIA 3-310 | 14.8±1.3 |
| 12 | BIA 3-315 | 15.4±1.8 |
| 13 | BIA 3-318 | 7.7±0.6 |
| 14 | BIA 3-320 | 8.5±0.3 |
| 15 | BIA 3-325 | 2.9±0.3 |
| 16 | BIA 3-329 | 3.2±0.2 |
| 17 | BIA 3-333 | 7.7±0.5 |
| 18 | BIA 3-335 | 4.2±0.2 |
| 19 | BIA 3-340 | 6.8±2.3 |
| 20 | BIA 3-343 | 3.6±0.3 |
| 21 | BIA 3-356 | 0.9±0.6 |
| 22 | BIA 3-358 | 0.0±0.0 |
| 23 | BIA 3-363 | 4.4±0.9 |
| 24 | BIA 3-373 | 0.0±0.0 |
| 25 | BIA 3-374 | 0.0±0.0 |
| 26 | BIA 3-383 | 4.8±0.4 |
| 27 | BIA 3-384 | 11.4±4.0 |
| 28 | BIA 3-409 | 10.0±2.6 |
| 29 | BIA 3-426 | 1.5±0.6 |
| 30 | BIA 3-427 | 1.9±1.3 |
| Entacapone | | 23.0±3.0 |

**Table 2.**

| COMT activity (% of control) in homogenates of mice liver and brain, determined at 1 and 6 h after administration by gastric tube of compounds 1- 30 and entacapone. Results are means±S.E.M. of 4-8 experiments per group. | | | | | |
|---|---|---|---|---|---|
| | | **Liver COMT (% of control)** | | **Brain COMT (% of control)** | |
| | Compounds | **1h** | **6h** | **1h** | **6h** |
| 1 | BIA 3-257 | 40.8±6.4 | 49.9±2.2 | 45.0±15.0 | 102.0±19.0 |
| 2 | BIA 3-260 | 24.6±4.8 | 56.9±17.7 | 80.0±11.0 | 95.0±12.0 |
| 3 | BIA 3-282 | 13.5±3.2 | 13.6±3.2 | 81.0±11.0 | 71.0±11.0 |
| 4 | BIA 3-283 | 12.0±1.8 | 14.4±4.3 | 55.0±8.7 | 49.6±4.9 |
| 5 | BIA 3-284 | 16.7±1.6 | 35.2±9.6 | 38.6±6.4 | 57.2±5.1 |
| 6 | BIA 3-286 | 57.6±5.8 | 99.9±10.3 | 37.0±3.3 | 66.0±2.6 |
| 7 | BIA 3-294 | 38.4±2.8 | 57.1±4.9 | 74.0±12.0 | 126.0±8.0 |
| 8 | BIA 3-303 | 22.0±3.7 | 49.6±9.4 | 61.0±4.0 | 104.4±6.2 |
| 9 | BIA 3-304 | 29.0±1.0 | 35.0±6.0 | 59.0±6.0 | 93.0±9.0 |
| 10 | BIA 3-309 | 17.3±2.7 | 38.7±4.7 | 83.0±5.0 | 119.0±13.0 |
| 11 | BIA 3-310 | 38.0±5.0 | 73.0±10.0 | 80.0±5.0 | 103.0±7.0 |
| 12 | BIA 3-315 | 33.0±2.0 | 52.0±13.0 | 32.0±2.0 | 84.0±6.0 |
| 13 | BIA 3-318 | 49.7±3.2 | 46.7±2.9 | 76.0±9.0 | 100.0±10.0 |
| 14 | BIA 3-320 | 33.6±5.6 | 50.3±5.6 | 95.4±11.4 | 121.2±11.8 |
| 15 | BIA 3-325 | 51.7±5.9 | 37.9±8.4 | 93.0±9.0 | 122.0±9.0 |
| 16 | BIA 3-329 | 22.1±3.4 | 71.7±21.6 | 25.0±13.0 | 95.0±12.0 |
| 17 | BIA 3-333 | 36.8±2.3 | 69.6±8.9 | 96.0±5.0 | 109.0±14.0 |
| 18 | BIA 3-335 | 18.5±6.8 | 26.3±3.9 | 86.0±14.0 | 86.0±11.0 |
| 19 | BIA 3-340 | 30.3±4.0 | 57.9±8.6 | 93.0±19.0 | 107.0±3.0 |
| 20 | BIA 3-343 | 39.0±2.0 | 64.0±12.0 | 106.0±9.0 | 125.0±5.0 |
| 21 | BIA 3-356 | 55.1±5.0 | 77.5±18.0 | 96.0±8.0 | 104.0±17.0 |
| 22 | BIA 3-358 | 54.2±7.5 | 71.7±0.4 | 94.0±3.0 | 96.0±18.0 |
| 23 | BIA 3-363 | 30.0±2.5 | 56.3±6.0 | 87.0±6.0 | 123.0±12.0 |
| 24 | BIA 3-373 | 16.5±1.5 | 37.7±3.9 | 33.0±8.0 | 98.0±2.0 |
| 25 | BIA 3-374 | 13.7±1.9 | 31.1±5.5 | 62.8±5.1 | 92.4±10.8 |
| 26 | BIA 3-383 | 18.3±2.9 | 55.2±4.7 | 69.8±3.2 | 89.6±13.4 |
| 27 | BIA 3-384 | ± | ± | ± | ± |
| 28 | BIA 3-409 | ± | ± | ± | ± |
| 29 | BIA 3-426 | ± | ± | ± | ± |
| 30 | BIA 3-427 | ± | ± | ± | ± |
| | Entacapone | ± | ± | ± | ± |

### Conclusion

Compounds of general formula I are very potent catechol-*O*-methyltransferase (COMT) inhibitors and have potentially valuable pharmaceutical properties in the treatment of some central and peripheral nervous system disorders where inhibition of *O*-methylation of catecholamines may be of therapeutical benefit, such as mood disorders, Parkinson's disease and parkinsonian disorders, gastrointestinal disturbances, edema formation states and hypertension. The possibility to use a long acting COMT inhibitor with limited activity in the brain, such as compounds **2, 3, 10** and **18,** opens new perspectives in the treatment of Parkinson' disease and parkinsonian disorders, gastrointestinal disturbances, edema formation states and hypertension, by improving selectivity of COMT inhibition in the periphery. This is particularly important when thinking of treating patients afflicted by Parkinson's disease and taking L-DOPA plus a peripheral AADC inhibitor, due to the possibility that COMT inhibitors which have easy access to the brain may cause excessive dopaminergic stimulation, thereby inducing dyskinesia and mental confusion in L-DOPA treated patients.

The invention disclosed herein is exemplified by the following examples of preparation, which should not be construed to limit the scope of the disclosure. Alternative pathways and analogous structures may be apparent to those skilled in the art.

### Example 1

### 1-(3,4-Dihydroxy-5-nitro-phenyl)-2-(naphthalen-1 -yloxy)-ethanone (compound 25, Table 1)

To a stirred solution of 1-naphthol (1.54 g, 10.69 mmol) in DMF (10 mL) at room temperature was added potassium carbonate (1.48 g, 10.69 mmol) in one portion followed by 2-chloro-1-(3,4-dihydroxy-5-nitro-phenyl)-ethanone (0.75 g, 3.24 mmol). The resulting deep red suspension was stirred at 100 °C for one hour and then allowed to cool to room temperature. The inorganic material was removed by filtration and the filter cake was washed by DMF (2 mL). The combined filtrate was then evaporated in vacuo and water (15 mL) was added to the residue. The mixture was extracted by ethyl acetate and the organic extracts were washed by water and then dried over anhydrous sodium sulphate. Filtration and concentration in vacuo afforded an orange/brown solid which was triturated with diethyl ether (10 mL). The light orange solid was filtered off and recrystallised from acetic acid to afford orange crystals of m.p. 198-199 °C.

### Examples 2-7

By the application of the above described technique and related procedures known to those skilled in the art and using the appropriate phenols, the following compounds were prepared:
1-(3,4-Dihydroxy-5-nitro-phenyl)-2-(naphthalen-2-yloxy)-ethanone (compound 10, Table 1)
1-(3,4-Dihydroxy-5-nitro-phenyl)-2-(3-methoxy-naphthalen-2-yloxy)-ethanone
1-(3,4-Dihydroxy-5-nitro-phenyl)-2-phenoxy-ethanone (compound 8, Table 1)
1-(3,4-Dihydroxy-5-nitro-phenyl)-2-(4-methoxy-phenoxy)-ethanone (compound 9, Table 1)
1-(3,4-Dihydroxy-5-nitro-phenyl)-2-(3,4-dimethyl-phenoxy)-ethanone
1-(3,4-Dihydroxy-5-nitro-phenyl)-2-(2-fluoro-phenoxy)-ethanone

### Example 8

### 1-(3,4-Dihydroxy-5-nitro-phenyl)-2-p-tolylsulphanyl-ethanone (compound 28, Table 1)

To a stirred solution of 4-thiocresol (0.19 g, 1.49 mmol) in DMF (4 mL) at room temperature was added potassium carbonate (0.21 g, 1.49 mmol) in one portion followed by 2-bromo-1-(3,4-dihydroxy-5-nitro-phenyl)-ethanone (0.41 g, 1.49 mmol) in portions. The resulting mixture was stirred for twenty minutes and then filtered. The filtrate was evaporated in vacuo and the residue was treated with water (5 mL) and extracted with ethyl acetate. The organic extracts were washed by 1N HCI, water and brine, then dried over anhydrous sodium sulphate. Filtration and evaporation in vacuo gave a yellow oil which crystallised on standing. Recrystallisation from acetic acid afforded yellow crystals of m.p. 115-116 °C.

### Example 9

By the application of the above described technique and related procedures known to those skilled in the art and using the appropriate thiophenol, the following compound was prepared:
1-(3,4-Dihydroxy-5-nitro-phenyl)-2-(4-methoxy-phenylsulphanyl)-ethanone

### Example 10

### 1-(3,4-Dihydroxy-5-nitro-phenyl)-3-p-tolylsulphanyl-propan-1-one

To a stirred solution of 4-thiocresol (0.071 g, 0.57 mmol) in DMF (2 mL) at room temperature was added potassium carbonate (0.079 g, 0.57 mmol) in one portion followed by 1-(3,4-dihydroxy-5-nitro-phenyl)-propenone (0.10 g, 0.48 mmol). The resulting red suspension was stirred at room temperature for twenty minutes and then filtered and acidified by addition of 2N HCI. The solvent was then removed in vacuo and water (5 mL) was added to the residue, which was extracted with ethyl acetate. The organic extracts were washed by water and brine, then dried over anhydrous sodium sulphate. Filtration and evaporation in vacuo afforded an orange/brown solid which was recrystallised from acetic acid to give orange crystals of m.p. 160-161 °C.

### Example 11

By the application of the above described technique and related procedures known to those skilled in the art and using the appropriate thiophenol, the following compound was prepared:
1-(3,4-Dihydroxy-5-nitro-phenyl)-3-(pyrimidin-2-ylsulphanyl)-propan-1-one

### Example 12

### 2-(4-Chloro-phenylamino)-1-(3,4-dihydroxy-5-nitro-phenyl)-ethanone

To a stirred solution of 2-chloro-1-(3,4-dihydroxy-5-nitro-phenyl)-ethanone (0.35 g, 1.50 mmol) in DMF (5 mL) at room temperature was added potassium iodide (0.25 g, 1.50 mmol) followed by 4-chloroaniline (0.49 g, 3.85 mmol). The resulting mixture was stirred for two hours and then poured onto water (10 mL) and extracted by ethyl acetate. The organic extracts were washed by 2N HCI, water and brine, then dried over anhydrous sodium sulphate. Filtration and evaporation in vacuo gave a brown solid that was triturated with diethyl ether (5 mL). The resulting dark orange solid was removed by filtration and recrystallised from acetic acid to afford orange crystals of m.p. 177-178 °C.

### Examples 13-15

By the application of the above described technique and related procedures known to those skilled in the art and using the appropriate anilines, the following compounds were prepared:
1-(3,4-Dihydroxy-5-nitro-phenyl)-2-phenylamino-ethanone
2-(4-Bromo-phenylamino)-1-(3,4-dihydroxy-5-nitro-phenyl)-ethanone
1-(3,4-Dihydroxy-5-nitro-phenyl)-2-(4-methoxy-phenylamino)-ethanone

### Example 16

### 1-(3,4-Dihydroxy-5-nitro-phenyl)-2-propylamino-propan-1-one hydrochloride

To a stirred solution of 1-(3,4-dihydroxy-5-nitro-phenyl)-propenone ( 0.10 g, 0.48 mmol) in DMF (1 mL) at room temperature was added dropwise propylamine (0.11 g, 1.92 mmol). The resulting deep red solution was stirred at room temperature for twenty minutes and then the solvent was removed in vacuo. The residue was taken up in ethanol (2 mL) and acidified with a few drops of concentrated hydrochloric acid. The solution was cooled and diluted with diethyl ether. The precipitate was filtered off and washed by diethyl ether (1 mL) to give orange crystals of m.p. 195-196 °C.

### Examples 17-20

By the application of the above described technique and related procedures known to those skilled in the art and using the appropriate primary amines, the following compounds were prepared:
2-Benzylamino-1-(3,4-dihydroxy-5-nitro-phenyl)-propan-1-one hydrochloride
3-Cyclohexylamino-1-(3,4-dihydroxy-5-nitro-phenyl)-propan-1-one hydrochloride
3-Cyclopentylamino-1-(3,4-dihydroxy-5-nitro-phenyl)-propan-1-one hydrochloride
1-(3,4-Dihydroxy-5-nitro-phenyl)-3-(piperidin-1 -ylamino)-propan-1 -one hydrochloride

### Example 21

### 1-(3,4-Dihydroxy-5-nitro-phenyl)-3-phenyl-3-piperidin-1-yl-propan-1-one hydrochloride (compound 3, Table 1)

To a stirred solution of 1-(3,4-dihydroxy-5-nitro-phenyl)-3-phenyl-propenone (0.14 g, 0.50 mmol) in DMF (0.7 mL) at room temperature was added p-toluenesulphonic acid (0.095 g, 0.50 mmol) followed by piperidine (0.25 mL, 2.5 mmol) and the mixture stirred for two hours. A 1N solution of HCI in ethanol (3 mL) was added followed by diisopropyl ether (3 mL). The precipitate was removed by filtration and washed by ethanol to give crystals of m.p. 177-178 °C.

### Examples 22-23

By the application of the above described technique and related procedures known to those skilled in the art and using the appropriate cyclic secondary amines, the following compounds were prepared:
1-(3,4-Dihydroxy-5-nitro-phenyl)-3-morpholin-4-yl-3-phenyl-propan-1-one hydrochloride (compound 5, Table 1)
1-(3,4-Dihydroxy-5-nitro-phenyl)-3-phenyl-3-pyrrolidin-1 -yl-propan-1-one hydrochloride (compound 4, Table 1)

### Example 24

### 1-(3,4-Dihydroxy-5-nitro-phenyl)-2-pyridinium ethanone chloride

To a solution of 2-chloro-1-(3,4-dihydroxy-5-nitro-phenyl)-ethanone (0.23 g, 0.99 mmol) in DMF (3 mL) at room temperature was added pyridine (0.31 g, 3.97 mmol) dropwise. After a few minutes an orange precipitate formed and the mixture was then warmed to 100 °C for one hour. After cooling to room temperature, the solvent was removed in vacuo and the residue taken up in ethanol (5 mL) and acidified with concentrated hydrochloric acid. The resulting orange solid was filtered off and washed with ethanol to give crystals of m.p. 285-287 °C.

### Example 25

### 2-[2-(3,4-Dihydroxy-5-n itro-phenyl)-2-oxo-ethyl]-isoindole-1,3-dione

To a stirred solution of phthalimide (8.14 g, 55.35 mmol) in DMF (30 mL) at room temperature was added potassium carbonate (7.64 g, 55.36 mmol) followed by 2-chloro-1-(3,4-dihydroxy-5-nitro-phenyl)-ethanone (4.0 g, 17.28 mmol) and the resulting suspension was warmed to 100 °C for one hour. After cooling to room temperature, the mixture was filtered and the filter cake washed by DMF (5 mL). The combined filtrate was concentrated in vacuo and the residue suspended in ethanol (20 mL) and acidified by the addition of concentrated hydrochloric acid. On cooling, crystals formed which were filtered off and recrystallised from acetic acid to afford dark yellow crystals of m.p. 214-216 °C.

### Example 26

By the application of the above described technique and related procedures known to those skilled in the art and using the appropriate halo-ketone, the following compound was prepared:
2-[2-(3,4-dihydroxy-5-nitro-phenyl)-3-oxo-propyl]-isoindole-1,3-dione

### Example 27 (Not an example of the invention)

### 2-Amino-1-(3,4-dihydroxy-5-nitro-phenyl)-ethanone hydrochloride

To a stirred suspension of 2-[2-(3,4-dihydroxy-5-nitro-phenyl)-2-oxo-ethyl]-isoindole-1,3-dione (2.40 g, 7.02 mmol) in methanol (25 mL) at room temperature was added hydrazine hydrate (2.10 g, 42.06 mmol). The resulting orange suspension was stirred at reflux for forty minutes and then allowed to cool to room temperature. The mixture was acidified with concentrated hydrochloric acid and heated at reflux for one hour. The mixture was again cooled to room temperature and the insoluble material was filtered off and washed by methanol (5 mL). The combined filtrate was concentrated in vacuo and the residue triturated with warm ethanol to give orange crystals of m.p. 190-192 °C.

### Example 28

### Acetic acid, 2-acetoxy-4-(2-acetylamino-acetyl)-6-nitro-phenyl ester

To a stirred suspension of 2-amino-1-(3,4-dihydroxy-5-nitro-phenyl)-ethanone hydrochloride (0.20 g, 0.80 mmol) and 4-dimethylaminopyridine (0.02 g) in dichloromethane (20 mL) at room temperature was added pyridine (0.64 g, 8.05 mmol) dropwise. The mixture was then cooled in an ice-water bath and acetyl chloride (0.63 g, 8.05 mmol) was added dropwise. After stirring at room temperature for four hours, the mixture was filtered and the filter cake washed by dichloromethane (2 mL). The combined filtrate was washed by 1N HCl, water and brine, then dried over anhydrous sodium sulphate. Filtration and concentration in vacuo afforded an oil which crystallised on standing. Recrystallisation from a dichloromethane/heptane mixture afforded off-white crystals of m.p. 121-124 °C.

### Example 29

### 2-Diethylamino-1-(3,4-dihydroxy-5-nitro-phenyl)-ethanone hydrochloride

To a stirred solution of 2-chloro-1-(3,4-dihydroxy-5-nitro-phenyl)-ethanone (0.12 g, 0.54 mmol) in DMF (2 mL) at room temperature was added diethylamine (0.23 g, 3.20 mmol) dropwise and the mixture stirred for 2 hours. The solvent was evaporated in vacuo and the residue was taken up in ethanol (3 mL) and acidified by the addition of a few drops of concentrated hydrochloric acid. The precipitate formed on cooling was filtered off and washed with ethanol (1 mL) to afford beige crystals of m.p. 186-188 °C.

### Examples 30-32

By the application of the above described technique and related procedures known to those skilled in the art and using the appropriate halo-ketones and secondary amines, the following compounds were prepared:
2-Dimethylamino-1-(3,4-dihydroxy-5-nitro-phenyl)-ethanone hydrochloride
2-Diethylamino-1-(3,4-dihydroxy-5-nitro-phenyl)-propan-1-one hydrochloride (compound 7, Table 1)
2-Dimethylamino-1 -(3,4-dihydroxy-5-nitro-phenyl)-propan-1 -one hydrochloride

### Example 33

### 1-(3,4-Dihydroxy-5-nitro-phenyl)-2-pyrrolidin-1-yl-ethanone hydrochloride

To a stirred solution of 2-chloro-1-(3,4-dihydroxy-5-nitro-phenyl)-ethanone (0.25 g, 1.08 mmol) in DMF (3 mL) at room temperature was added pyrrolidine (0.27 g, 3.78 mmol) dropwise and the mixture was stirred for two hours. The solvent was then removed in vacuo and the residue was taken up in ethanol (2 mL) and acidified by the addition of a few drops of concentrated hydrochloric acid. The precipitate formed on cooling was filtered off and washed by ethanol (1 mL) to afford orange crystals of m.p. 213-214 °C

### Examples 34-64

By the application of the above described technique and related procedures known to those skilled in the art and using the appropriate halo-ketones and cyclic secondary amines, the following compounds were prepared:
1-(3,4-Dihydroxy-5-nitro-phenyl)-2-morpholin-4-yl-ethanone hydrochloride
1-(3,4-Dihydroxy-5-nitro-phenyl)-2-piperidin-1-yl-ethanone hydrochloride
2-Azepan-1-yl-1-(3,4-dihydroxy-5-nitro-phenyl)-ethanone hydrochloride (compound 1, Table 1)
1-(3,4-Dihydroxy-5-nitro-phenyl)-2-thiomorpholin-4-yl-ethanone hydrochloride
1-(3,4-Dihydroxy-5-nitro-phenyl)-2-(4-methyl-piperidin-1-yl)-ethanone hydrochloride
1-(3,4-Dihydroxy-5-nitro-phenyl)-2-(3-methyl-piperidin-1-yl)-ethanone hydrochloride
1-(3,4-Dihydroxy-5-nitro-phenyl)-2-(2-methyl-piperidin-1-yl)-ethanone hydrochloride (compound 6, Table 1)
1-(3,4-Dihydroxy-5-nitro-phenyl)-2-(2,6-dimethyl-morpholin-4-yl)-ethanone hydrochloride (compound 11, Table 1)
2-[1,4']Bipiperidinyl-1 '-yl-1-(3,4-dihydroxy-5-nitro-phenyl)-ethanone dihydrochloride
2-Azocan-1-yl-1-(3,4-dihydroxy-5-nitro-phenyl)-ethanone hydrochloride
1-(3,4-Dihydroxy-5-nitro-phenyl)-2-(1,4-dioxa-8-aza-spiro[4.5]dec-8-yl)-ethanone hydrochloride
1-(3,4-Dihydroxy-5-nitro-phenyl)-2-(3,5-dimethyl-piperidin-1-yl)-ethanone hydrochloride (compound 12, Table 1)
1-(3,4-Dihydroxy-5-nitro-phenyl)-2-imidazol-1-yl-ethanone hydrochloride
2-(3,4-Dihydro-1*H*-isoquinolin-2-yl)-1-(3,4-dihydroxy-5-nitro-phenyl)-ethanone hydrochloride (compound 21, Table 1)
1-[2-(3,4-Dihydroxy-5-nitro-phenyl)-2-oxo-ethyl]-piperidine-4-carboxylic acid amide hydrochloride
1-(3,4-Dihydroxy-5-nitro-phenyl)-3-morpholin-4-yl-propan-1-one hydrochloride
1-(3,4-Dihydroxy-5-nitro-phenyl)-3-piperidin-1-yl-propan-1-one hydrochloride (compound 2, Table 1)
1-(3,4-Dihydroxy-5-nitro-phenyl)-3-(2,6-dimethyl-morpholin-4-yl)-propan-1-one hydrochloride
3-[1,4']Bipiperidinyl-1'yl-1-(3,4-dihydroxy-5-nitro-phenyl)-propan-1-one dihydrochloride
1-(3,4-Dihydroxy-5-nitro-phenyl)-3-(1,4-dioxa-8-aza-spiro[4.5]dec-8-yl)-propan-1-one hydrochloride
3-Azocan-1-yl-1-(3,4-dihydroxy-5-nitro-phenyl)-propan-1-one hydrochloride (compound 16, Table 1)
1 -(3,4-Dihydroxy-5-nitro-phenyl)-3-(3,5-dimethyl-piperidin-1-yl)-propan-1-one hydrochloride
1-[3-(3,4-Dihydroxy-5-nitro-phenyl)-3-oxo-propyl]-piperidine-4-carboxylic acid ethyl ester hydrochloride (compound 17, Table 1)
1-(3,4-Dihydroxy-5-nitro-phenyl)-3-thiomorpholin-4-yl-propan-1-one hydrochloride (compound 19, Table 1)
1-(3,4-Dihydroxy-5-nitro-phenyl)-3-pyrrolidin-1-yl-propan-1-one hydrochloride
3-Azepan-1-yl-1-(3,4-dihydroxy-5-nitro-phenyl)-propan-1-one hydrochloride (compound 20, Table 1)
3-(5,6-Dihydro-4*H*-pyrimidin-1 -yl)-1 -(3,4-dihydroxy-5-nitro-phenyl)-propan-1-one hydrochloride
1-(3,4-Dihydroxy-5-nitro-phenyl)-3-thiazolidin-3-yl-propan-1-one hydrochloride
1-(3,4-Dihydroxy-5-nitro-phenyl)-3-(4-methyl-piperidin-1 -yl)-propan-1 -one hydrochloride
1-(3,4-Dihydroxy-5-nitro-phenyl)-3-(3-methyl-piperidin-1-yl)-propan-1-one hydrochloride
1-(3,4-Dihydroxy-5-nitro-phenyl)-3-(2-methyl-piperidin-1-yl)-propan-1-one hydrochloride (compound 26, Table 1)

### Example 65

### 3-(4-Benzyl-piperidin-1-yl)-1-(3,4-dihydroxy-5-nitro-phenyl)-propan-1-one hydrochloride (compound 24, Table 1)

To a stirred suspension of 1-(3,4-dihydroxy-5-nitro-phenyl)-ethanone (0.2 g, 1.02 mmol) in isopropanol (5 mL) at room temperature was added 4-benzylpiperidine (0.71 g, 4.06 mmol) followed by 35% aqueous formaldehyde solution (0.5 mL, 5.08 mol) and concentrated hydrochloric acid (0.42 mL, 6.10 mmol). The resulting mixture was heated at reflux for six hours, during which time a precipitate formed. The mixture was allowed to cool to room temperature and the precipitate was filtered off and washed with ethanol (2 mL) to give yellow crystals of m.p. 186-188°C

### Example 66-68

By the application of the above described technique and related procedures known to those skilled in the art and using the appropriate cyclic secondary amines, the following compounds were prepared:
3-(3,4-Dihydro-1*H*-isoquinolin-2-yl)-1-(3,4-dihydroxy-5-nitro-phenyl)-propan-1-one hydrochloride (compound 27, Table 1)
1-[3-(3,4-Dihydroxy-5-nitro-phenyl)-3-oxo-propyl]-4-phenyl-piperidine-4-carbonitrile hydrochloride (compound 15, Table 1)
1-[3-(3,4-Dihydroxy-5-nitro-phenyl)-3-oxo-propyl]-piperidine-4-carboxylic acid diethylamide hydrochloride

### Example 69

### 2-(4-Benzyl-piperazin-1-yl)-1-(3,4-dihydroxy-5-nitro-phenyl)-ethanone dihydrochloride

To a stirred solution of 2-chloro-1-(3,4-dihydroxy-5-nitro-phenyl)-ethanone (0.25 g, 1.08 mmol) in DMF (3 mL) at room temperature was added 4-benzylpiperidine (0.66 g, 3.78 mmol) and the resulting red solution was stirred for two hours. The solvent was then removed in vacuo and the residue was taken up in ethanol (3 mL) and acidified by the addition of a few drops of concentrated hydrochloric acid. After cooling, the resulting precipitate was filtered off and washed with ethanol (1 mL) to give orange crystals of m.p. 178-180 °C

### Examples 70-88

By the application of the above described technique and related procedures known to those skilled in the art and using the appropriate halo-ketones and substituted piperazines, the following compounds were prepared:
2-[4-(3,4-Dichloro-phenyl)-piperazin-1-yl]-1-(3,4-dihydroxy-5-nitro-phenyl)-ethanone dihydrochloride
1-(3,4-Dihydroxy-5-nitro-phenyl)-2-[4-(2-methoxy-phenyl)-piperazin-1-yl]-ethanone dihydrochloride
1-(3,4-Dihydroxy-5-nitro-phenyl)-2-(4-methyl-piperazin-1-yl)-ethanone dihydrochloride
1-(3,4-Dihydroxy-5-nitro-phenyl)-2-(4-pyridin-2-yl-piperazin-1-yl)-ethanone trihydrochloride (compound 14, Table 1)
1-(3,4-Dihydroxy-5-nitro-phenyl)-2-[4-(4-fluoro-phenyl)-piperazin-1-yl]-ethanone dihydrochloride
1-(3,4-Dihydroxy-5-nitro-phenyl)-2-[4-(2-hydroxy-ethyl)-piperazin-1-yl]-ethanone dihydrochloride
4-[2-(3,4-Dihydroxy-5-nitro-phenyl)-2-oxo-ethyl]-piperazine-1-carboxylic acid ethyl ester dihydrochloride
2-(4-Benzo[1,3]dioxol-5-ylmethyl-piperazin-1-yl)-1-(3,4-dihydroxy-5-nitro-phenyl)-ethanone dihydrochloride
1 -(3,4-Dihydroxy-5-nitro-phenyl)-2-(4-ethyl-piperazin-1 -yl)-ethanone dihydrochloride
1-(3,4-Dihydroxy-5-nitro-phenyl)-2-[4-(3-trifluoromethyl-phenyl)-piperazin-1-yl]-ethanone dihydrochloride (compound 13, Table 1)
2-[4-(4-Acetyl-phenyl)-piperazin-1-yl]-1-(3,4-dihydroxy-5-nitro-phenyl)-ethanone dihydrochloride
1-(3,4-Dihydroxy-5-nitro-phenyl)-2-(4-phenyl-piperazin-1-yl)-ethanone dihydrochloride
1-(3,4-Dihydroxy-5-nitro-phenyl)-3-(4-methyl-piperazin-1 -yl)-propan-1-one dihydrochloride
1-(3,4-Dihydroxy-5-nitro-phenyl)-3-(4-ethyl-piperazin-1-yl)-propan-1-one dihydrochloride
4-[3-(3,4-Dihydroxy-5-nitro-phenyl)-2-oxo-propyl]-piperazine-1-carboxylic acid ethyl ester dihydrochloride
1-(3,4-Dihydroxy-5-nitro-phenyl)-3-[4-(4-fluoro-phenyl)-piperazin-1-yl]-propan-1-one dihydrochloride
1-(3,4-Dihydroxy-5-nitro-phenyl)-3-[4-(3-trifluoromethyl-phenyl)-piperazin-1-yl]-propan-1-one dihydrochloride (compound 18, Table 1)
1 -(3,4-Dihydroxy-5-nitro-phenyl)-3-(4-phenyl-piperazin-1-yl)-propan-1 -one dihydrochloride
1-(3,4-Dihydroxy-5-nitro-phenyl)-3-[4-(4-nitro-phenyl)-piperazin-1-yl]-propan-1-one dihydrochloride

### Example 89

### 3-[4-(2-Chloro-phenyl)-piperazin-1-yl]-1-(3,4-dihydroxy-5-nitro-phenyl)-propan-1-one dihydrochloride (compound 22, Table 1)

A stirred solution of 1-(3,4-dihydroxy-5-nitro-phenyl)-ethanone ( 0.20 g, 1.02 mmol) in ispropanol (5 mL) was treated with 2-(chlorophenyl)-piperazine hydrochloride (0.95 g, 4.06 mmol), 35% aqueous formaldehyde solution (0.5 mL, 5.08 mmol) and concentrated hydrochloric acid (0.42 mL, 6.10 mmol) and the mixture was heated at reflux for six hours, during which time a precipitate formed. After cooling to room temperature, the precipitate was filtered off and washed with ethanol (2 mL) to afford yellow crystals of m.p. 214-216 °C.

### Examples 90-96

By the application of the above described technique and related procedures known to those skilled in the art and using the appropriate substituted piperazines, the following compounds were prepared:
3-[4-(4-Chloro-phenyl)-piperazin-1-yl]-1-(3,4-dihydroxy-5-nitro-phenyl)-propan-1-one dihydrochloride
1-(3,4-Dihydroxy-5-nitro-phenyl)-3-[4-(2-methyl-phenyl)-piperazin-1-yl]-1-propan-1-one dihydrochloride
1-(3,4-Dihydroxy-5-nitro-phenyl)-3-(4-pyrimidin-2-yl-piperazin-1-yl)-propan-1-one tetrahydrochloride (compound 23, Table 1)
1 -(3,4-Dihydroxy-5-nitro-phenyl)-3-[4-(4-methoxy-phenyl)-piperazin-1-yl]-1-propan-1-one dihydrochloride
1-(3,4-Dihydroxy-5-nitro-phenyl)-3-(4-propyl-piperazin-1-yl)-propan-1-one dihydrochloride
3-[4-(3,4-Dichloro-phenyl)-piperazin-1-yl]-1-(3,4-dihydroxy-5-nitro-phenyl)-propan-1-one dihydrochloride
1-(3,4-Dihydroxy-5-nitro-phenyl)-3-[4-(2-methoxy-phenyl)-piperazin-1-yl]-1-propan-1-one dihydrochloride

### Example 97

### 3-(4-Benzoyl-piperazin-1-yl)-1-(3,4-dihydroxy-5-nitro-phenyl)-propan-1-one dihydrochloride (compound 29, Table 1)

To a stirred solution of 1-(3,4-dihydroxy-5-nitro-phenyl)-3-phenyl-propenone (0.05 g, 0.24 mmol) in DMF (1 mL) at room temperature was added 4-benzoylpiperazine (0.15 g, 0.78 mmol) and the resulting red solution left stirring for one hour, whereupon the solvent was removed in vacuo. Ethanol (1 mL) was added to the residue which was acidified by the addition of a few drops of concentrated hydrochloric acid. The precipitate which formed on cooling was filtered off and washed by ethanol (0.5 mL) to afford yellow crystals of m.p. 201-203 °C.

### Examples 98-99

By the application of the above described technique and related procedures known to those skilled in the art and using the appropriate substituted benzoylpiperazines, the following compounds were prepared:
1-(3,4-Dihydroxy-5-nitro-phenyl)-3-[4-(4-methoxy-benzoyl)-piperazin-1-yl]-propan-1-one dihydrochloride (compound 30, Table 1)
1-(3,4-Dihydroxy-5-nitro-phenyl)-3-[4-(4-nitro-benzoyl)-piperazin-1-yl]-propan-1-one dihydrochloride

## Claims

1. A compound of formula I: 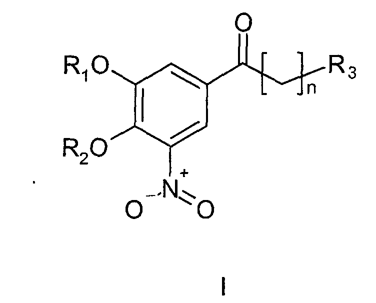 where R₁ and R₂ are the same or different and signify hydrogens or groups hydrolysable under physiological conditions, optionally substituted lower alkanoyl or aroyl, optionally substituted lower alkyl or arylsulphonyl or optionally substituted lower alkylcarbamoyl or taken together signify a lower alkylidene or cycloalkylidene group; n signifies the number 1 or 2; R₃ represents the group -O-R₄ wherein R₄ signifies an aryl group or R₃ represents the group -S-R₅ wherein R₅ signifies an aryl or heteroaryl group; or R₃ represents the group -NH-R₆ wherein R₆ signifies alkyl, cycloalkyl, heterocycloalkyl, alkylaryl, aryl, heteroaryl or alkylcarbonyl group; or R₃ represents the group -CHR₄NR₇ wherein NR₇ signifies a heterocycloalkyl group connected via the ring nitrogen atom; or R₃ represents the group NR₈R₉ where R₈ and R₉ signify alkyl groups, or taken together form a pyridyl, phthalimido or a heterocycloalkyl group connected via the ring nitrogen atom; or R₃ signifies a piperidine or piperazine ring optionally substituted by alkyl, alkylaryl, heterocycloalkyl, aryl, heteroaryl, alkyloxy, alkoxycarbonyl, alkylcarbonyl, cyano, carbamoyl or N-lower alkyl substituted or disubstituted carbamoyl group; the term alkyl means carbon chains, straight or branched, containing from one to six carbon atoms, optionally substituted by alkoxy, halogen, alkoxycarbonyl or hydroxycarbonyl groups; the term cycloalkyl represents an alicyclic group with three to six carbon atoms; the term aryl means a phenyl or naphthyl group, optionally substituted by alkoxy, halogen, or nitro groups; the term heteroaryl represents a five or six-membered ring, incorporating an atom of oxygen, sulphur or nitrogen; the term heterocycloalkyl represents a four to eight-membered cyclic ring optionally incorporating other atoms of oxygen, sulphur or nitrogen; the term halogen represents fluorine, chlorine, bromine or iodine and to the pharmacologically acceptable salts thereof.

2. A compound according to claim 1, comprising: 2-azepan-1-yl-1-(3,4-dihydroxy-5-nitro-phenyl)-ethanone hydrochloride; 1-(3,4-dihydroxy-5-nitro-phenyl)-3-piperidin-1-yl-propan-1-one hydrochloride; 1-(3,4-dihydroxy-5-nitro-phenyl)-3-phenyl-3-piperidin-1-yl-propan-1-one hydrochloride; 1-(3,4-dihydroxy-5-nitro-phenyl)-3-phenyl-3-pyrrolidin-1-yl-propan-1-one hydrochloride; 1-(3,4-dihydroxy-5-nitro-phenyl)-3-morpholin-4-yl-3-phenyl-propan-1-one hydrochloride; 2-diethylamino-1-(3,4-dihydroxy-5-nitro-phenyl)-propan-1-one hydrochloride; 1-(3,4-dihydroxy-5-nitro-phenyl)-2-phenoxy-ethanone; 1-(3,4-dihydroxy-5-nitro-phenyl)-2-(4-methoxy-phenoxy)-ethanone; 1-(3,4-dihydroxy-5-nitro-phenyl)-2-(naphthalen-2-yloxy)-ethanone; 1-(3,4-dihydroxy-5-nitro-phenyl)-2-(2,6-dimethyl-morpholin-4-yl)-ethanone hydrochloride; 1-(3,4-dihydroxy-5-nitro-phenyl)-2-(3,5-dimethyl-piperidin-1-yl-ethanone hydrochloride; 1-(3,4-dihydroxy-5-nitro-phenyl)-2-[4-(3-trifluoromethyl-phenyl)-piperazin-1-yl]-ethanone dihydrochloride; 1-(3,4-dihydroxy-5-nitro-phenyl)-2-(4-pyridin-2-yl-piperazin-1-yl)-ethanone trihydrochloride; 3-azocan-1-yl-1-(3,4-dihydroxy-5-nitro-phenyl)-propan-1-one hydrochloride; 1-[3-(3,4-dihydroxy-5-nitro-phenyl)-3-oxo-propyl]-piperidine-4-carboxylic acid ethyl ester hydrochloride; 1-(3,4-dihydroxy-5-nitro-phenyl)-3-[4-(3-trifluoromethyl-phenyl)-piperazin-1-yl]-propan-1-one dihydrochloride; 1-(3,4-dihydroxy-5-nitro-phenyl)-3-thiomorpholin-4-yl-propan-1-one hydrochloride; 3-azepan-1-yl-1-(3,4-dihydroxy-5-nitro-phenyl)-propan-1-one hydrochloride; 2-(3,4-dihydro-1*H*-isoquinolin-2-yl)-1-(3,4-dihydroxy-5-nitro-phenyl)-ethanone hydrochloride; 3-[4-(2-chloro-phenyl)-piperazin-1-yl]-1-(3,4-dihydroxy-5-nitro-phenyl)-propan-1-one dihydrochloride; 1-(3,4-dihydroxy-5-nitro-phenyl)-3-(4-pyrimidin-2-yl-piperazin-1-yl)-propan-1-one tetrahydrochloride; 3-(4-benzyl-piperidin-1-yl)-1-(3,4-dihydroxy-5-nitro-phenyl)-propan-1-one hydrochloride; 1-(3,4-dihydroxy-5-nitro-phenyl)-2-(naphthalen-1-yloxy)-ethanone; 1-(3,4-dihydroxy-5-nitro-phenyl)-3-(2-methyl-piperidin-1-yl)-propan-1-one hydrochloride; 1-(3,4-dihydroxy-5-nitro-phenyl)-2-p-tolylsulphanyl-ethanone; 3-(4-benzoyl-piperazin-1-yl)-1-(3,4-dihydroxy-5-nitro-phenyl)-propan-1-one dihydrochloride or 1-(3,4-dihydroxy-5-nitro-phenyl)-3-[4-(4-methoxy-benzoyl)-piperazin-1-yl]-propan-1-one dihydrochloride.

3. A method of treating a subject afflicted by some central and peripheral nervous system disorders, where a reduction in the *O*-methylation of catecholamines may be of therapeutical benefit, such as mood disorders, Parkinson's disease and parkinsonian disorders, gastrointestinal disturbances, edema formation states and hypertension, which comprises administering to the subject an amount of a compound according to claim **1** or **2** effective to treat said diseases in the subject.

4. A pharmaceutical composition comprising a therapeutically effective amount of a compound according to claim **1** or **2** in combination with a pharmaceutically acceptable carrier.

5. The use of a compound according to claim **1** or **2** in the manufacture of a medication for treating a subject afflicted by central or peripheral nervous system disorders.

6. The use of a compound according to claim **1** or **2** in the manufacture of a medication for treating mood disorders, Parkinson's disease and parkinsonian disorders, gastrointestinal disturbances, edema formation states and hypertension.

7. The use of a compound according to claim **1** or **2** in therapy.

8. The use of a compound according to claim 1 or 2, for use in the manufacture of a medicament for use as a COMT inhibitor.
